# DEMANDE DE BREVET EUROPEEN

(11) **EP 1 238 655 A1**
(43) Date de publication de la demande: **11.09.2002**
(21) Numéro de dépôt: 02290454.4
(22) Date de dépôt: 25.02.2002
(51) Int. Cl.: A61K 7/48

(54) **Composition comprenant un dérivé de polyaminoacides, et utilisation de ce composé pour lutter contre les rides de la peau**

(30) Priorité: 05.03.2001 FR 0102979
(71) Demandeur: L'OREAL, 75008 Paris (FR)
(72) Inventeur: Philippe, Michel, 91320 Wissous (FR); Benard, Sylvie, 95570 Attainville (FR)
(74) Mandataire: Dodin, Catherine

(57) **Abrégé**

L'invention concerne une composition antirides comprenant, dans un milieu physiologiquement acceptable, au moins un composé de type dérivé de polyaminoacides, ainsi que l'utilisation de ce composé pour diminuer, effacer et/ou lisser les rides et/ou les ridules de la peau.

L'invention concerne également l'utilisation de ce composé comme agent tenseur pour diminuer, lisser et/ou effacer les rides et/ou les ridules de la peau.

## Description

La présente invention a pour objet l'utilisation de dérivés de polyaminoacides dans une composition notamment cosmétique ou dermatologique, destinée à être appliquée sur la peau ou les muqueuses, en particulier sur le visage, notamment pour le traitement, c'est-à-dire la diminution, l'effacement et/ou le lissage des rides et/ou ridules de la peau des êtres humains.

Au cours du processus de vieillissement, il apparaît différents signes caractéristiques sur la peau, se traduisant notamment par une modification de la structure et des fonctions cutanées. Les principaux signes cliniques du vieillissement cutané sont notamment l'apparition de ridules et/ou de rides, en augmentation avec l'âge. On constate en particulier une désorganisation du "grain" de la peau, c'est-à-dire que le micro-relief est moins régulier et présente un caractère anisotrope.

Il est connu de traiter ces signes du vieillissement en utilisant des compositions cosmétiques ou dermatologiques contenant des actifs capables de lutter contre le vieillissement, tels que les α-hydroxyacides, les β-hydroxyacides et les rétinoïdes. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat, conduisant rapidement à un lissage des rides et/ou ridules et à la disparition, même temporaire, des marques de fatigue.

Il a alors notamment été proposé d'utiliser une dispersion aqueuse de particules de polymère comme agent tenseur de la peau, conduisant à un camouflage des rides par lissage de la peau. Toutefois, ces compositions se présentent toujours sous forme aqueuse, ce qui peut entraîner, d'une part, des problèmes bactériologiques, et surtout, d'autre part, un démaquillage trop aisé en présence d'eau. En effet, il n'est pas possible, avec cette solution, de préparer une composition présentant une bonne rémanence à l'eau.

La demanderesse a constaté que, de façon surprenante et inattendue, l'utilisation de polymères bien particuliers, pouvait permettre l'obtention d'une composition susceptible d'être appliquée sur la peau et qui pouvait permettre d'améliorer le 'camouflage' et/ou d'estomper les rides et/ou ridules déjà formées, cet effet étant obtenu de façon immédiate.
En effet, les polymères mis en oeuvre dans la présente invention constituent des agents tenseurs particulièrement efficaces. On entend par « agent tenseur » des composés susceptibles d'avoir un effet tenseur, c'est-à-dire pouvant tendre la peau et, par cet effet de tension, lisser la peau et y faire diminuer voire disparaître de façon immédiate les rides et les ridules.

L'invention a donc pour objet une composition antirides comprenant, dans un milieu physiologiquement acceptable, au moins un composé de formule (I) tel que ci-après défini.
Un autre objet de l'invention est l'utilisation d'au moins un composé de formule (I) ou d'une composition cosmétique le comprenant, pour diminuer, effacer et/ou lisser les rides et/ou les ridules de la peau.
Un autre objet de l'invention est l'utilisation d'au moins un composé de formule (I) pour la fabrication d'une composition pharmaceutique destinée à diminuer, effacer et/ou lisser les rides et/ou les ridules de la peau.
Un autre objet de l'invention est l'utilisation d'au moins un composé de formule (I) ou d'une composition le comprenant, comme agent tenseur pour diminuer, lisser et/ou effacer les rides et/ou les ridules de la peau.
Un autre objet de l'invention est un procédé de traitement cosmétique d'une peau ridée consistant à appliquer sur la ride et/ou la ridule, une composition comprenant au moins un composé de formule (I).

On a constaté que les composés selon l'invention présentent un très bon pouvoir tenseur de la peau. Les compositions selon l'invention sont facilement applicables et s'étalent aisément. Elles permettent d'estomper immédiatement après application les rides et les ridules à la surface de la peau.
Les compositions de l'invention peuvent notamment être appliquées sur le visage et/ou sur le cou, notamment sur le décolleté.

Par ailleurs, la synthèse de ces composés est très rapide, et aisément industrialisable. Ces composés sont préparés à partir d'aminoacides bien définis, conduisant à des oligomères et polymères à haute reproductibilité. Ceci est un grand avantage par rapport aux produits naturels pour lesquels la définition et la reproductibilité des lots sont très délicates à maîtriser.
Les compositions selon l'invention présentent une texture légère et sont très confortables à porter tout au long de la journée. Elles permettent l'obtention d'un film de très bonne tenue, mou, souple, élastique et flexible sur la peau; il suit les mouvements du support sur lequel il est déposé, sans se craqueler et/ou se décoller. Il adhère notamment parfaitement sur la peau du visage.

Les composés employés dans la présente invention sont donc des homopolymères répondant à la formule (I) : 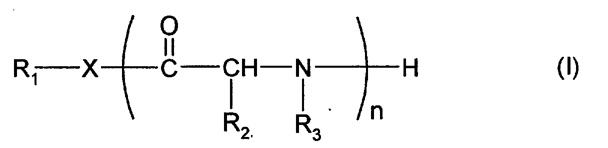 dans laquelle :
- X est choisi parmi -O-, -S- ou -NR, avec R représentant un atome d'hydrogène ou un radical hydrocarboné, linéaire ou ramifié, saturé ou insaturé en C₁-C₆,
- R₁ est choisi parmi :
   (i) un atome d'hydrogène,
   (ii) un radical hydrocarboné, linéaire ou ramifié, saturé ou insaturé, en C₁-C₄₀, éventuellement substitué par au moins un hydroxy ou un radical -NR'R", dans lequel R' et R" représentent, indépendamment l'un de l'autre, un atome d'hydrogène ou un radical hydrocarboné, linéaire ou ramifié, saturé ou insaturé en C₁-C₆; et/ou éventuellement interrompu par au moins un hétéroatome choisi parmi N, O ou Si,
   (iii) un radical choisi parmi 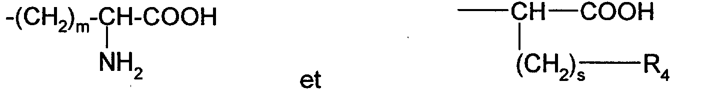 dans lesquels :
      - m est 1, 2, 3, 4 ou 5;
      - s est un entier compris entre 0 et 4 inclus;
      - R₄ représente un atome d'hydrogène, -NH₂, -OH, -SH, -CHOHCH₃, -CONH₂, 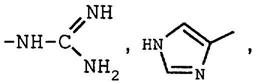 -C₆H₅ ou -C₆H₅pOH,
- R₂ représente un atome d'hydrogène, un radical hydrocarboné, linéaire ou ramifié, saturé ou insaturé, en C₁-C₈, ou un radical choisi parmi -CH₂OH, -CHOH-CH₃ , -CH₂C₆H₅, -CH₂C₆H₄p-OH et -(CH₂)ₜ-NH₂, avec t étant 1, 2, 3, 4 ou 5;
- R₃ représente un atome d'hydrogène ou un radical hydrocarboné, linéaire ou ramifié, saturé ou insaturé, en C₁-C₆,
- n est un nombre moyen d'unités répétitives supérieur à 1, et tel que le poids moléculaire du dérivé de polyaminoacide est compris entre 200 et 200.000, l'unité répétitive étant soit identique pour un même composé, soit différente, R₂ et/ou R₃ prenant alors au moins l'une des autres significations données pour ces radicaux.

Les composés de formule (I) peuvent également se présenter sous forme de sels minéraux ou organiques, compatibles avec une application dans les domaines cosmétique ou pharmaceutique.

De préférence, X représente O, S ou N-CH₃.

De préférence, R₁ représente un atome d'hydrogène; un radical hydrocarboné, linéaire ou ramifié, saturé ou insaturé, en C₁-C₂₂, notamment en C4-20, ou un radical choisi parmi : 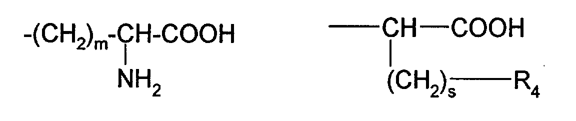 dans lesquels m, s et R4 ont les significations données ci-dessus.

De préférence, R₂ représente un atome d'hydrogène, un radical hydrocarboné, linéaire ou ramifié, saturé ou insaturé, en C₁-C₆, ou un radical -CH₂C₆H₄p-OH.

De préférence, R₃ représente un atome d'hydrogène ou un radical hydrocarboné, linéaire ou ramifié, saturé ou insaturé, en C₁-C₄, notamment méthyle ou éthyle.

En particulier, on peut citer les composés de formule (I) dans lesquels le radical R₁ représente l'une des formules suivantes :

C₁₅H₃₁-CH(OH)-CH(CH₂OH)-

C₁₀H₂₁-CH(C₈H₁₇)-CH₂-

C₁₆H₃₃-

C₈H₁₇-CH=CH-C₈H₁₆-

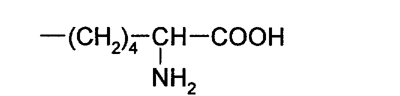

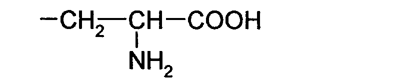

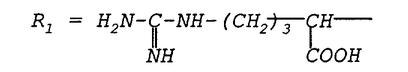

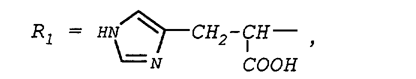

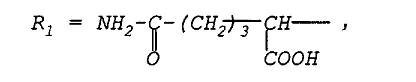

CH₂(OH)-CH(OH)-CH(OH)-CH(OH)-CH(OH)-CH₂-

De préférence, n est compris entre 3 et 500 et/ou est tel que le poids moléculaire du dérivé de polyaminoacide est compris entre 300 et 50 000.

Les dérivés de polyaminoacides de formule (I) peuvent être obtenus par des procédés bien connus de l'homme du métier, et notamment grâce à une réaction de polycondensation entre au moins un N-carboxyanhydride de formule : 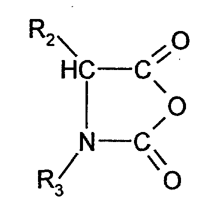 et un composé nucléophile de formule R₁-XH dans laquelle R₁, R₂, R₃ et X ont les mêmes significations que celles données ci-dessus pour la formule (I).
Ce procédé est notamment décrit dans la demande française FR2776510.

Les dérivés de polyaminoacide peuvent être utilisés, seuls ou en mélange, en une quantité de 0,001 à 30% en poids par rapport au poids total de la composition, de préférence en une quantité de 0,01 à 15% en poids par rapport au poids total de la composition.

Les composés selon l'invention peuvent recevoir des applications diverses, notamment dans des compositions cosmétiques ou pharmaceutiques, qui comprennent alors un milieu physiologiquement acceptable, notamment cosmétiquement ou pharmaceutiquement acceptable.

Ce milieu, ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux.

Lorsque la composition comprend une phase aqueuse, ladite phase peut comprendre de l'eau, une eau florale et/ou une eau minérale.
Ladite phase peut comprendre en outre des alcools tels que des monoalcools en C₁-C₆ et/ou des polyols tels que le glycérol, le butylèneglycol, l'isoprène glycol, le propylèneglycol, le polyéthylèneglycol.

La composition peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.
Les huiles volatiles sont généralement des huiles ayant, à 25°C, une tension de vapeur saturante au moins égale à 0,5 millibar (soit 50 Pa).
Parmi les constituants de la phase grasse, on peut citer :
- les silicones volatiles cycliques ayant de 3 à 8 atomes de silicium, de préférence de 4 à 6;
- les cyclocopolymères du type diméthylsiloxane/méthylalkylsiloxane,
- les silicones volatiles linéaires ayant de 2 à 9 atomes de silicium;
- les huiles volatiles hydrocarbonées, telles que les isoparaffines et notamment l'isododécane et des huiles fluorées;
- les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R1COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.
- les gommes de silicones;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

La composition peut comprendre en outre tout additif usuellement utilisé dans le domaine d'application envisagé, tel que des tensioactifs, des antioxydants, des parfums, des huiles essentielles, des conservateurs, des actifs cosmétiques ou pharmaceutiques, des vitamines, des acides gras essentiels, des sphingolipides, des agents autobronzants, des filtres solaires, des polymères filmogènes, des épaississants, des gélifiants, des colorants, des pigments, des charges, des nacres. Bien entendu l'homme du métier veillera à choisir les éventuels composés complémentaires et leur quantité, de manière telle que les propriétés avantageuses de la composition selon l'invention ne soient pas, ou substantiellement pas, altérées par l'adjonction envisagée.

La composition de l'invention peut se présenter sous toutes les formes galéniques normalement utilisées pour une application topique, notamment sous forme d'une solution aqueuse, hydroalcoolique ou huileuse; d'une émulsion huile-dans-eau, eau-dans-huile ou multiple, de consistance liquide ou semi-liquide du type lait, ou de consistance molle, semi-solide ou solide du type crème; d'un gel aqueux ou huileux; d'un produit anhydre liquide, pâteux ou solide; de dispersions aqueuses, huileuses ou en milieu solvant de type lotion ou sérum; de microémulsions; de microcapsules; de microparticules ou de dispersions vésiculaires de type ionique ou non ionique; sous forme fluide, épaissie ou gélifiée, semi-solide, pâte souple; sous forme solide telle que de stick ou bâton.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau.

Lorsque la composition de l'invention est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition.

En outre, on peut aussi associer aux agents tenseurs utilisés selon l'invention d'autres composés connus par l'homme du métier comme agents tenseurs ayant des propriétés différentes de celles des agents utilisés selon l'invention, notamment une protéine ou hydrolysat de protéine. Comme composés de ce type, on peut citer par exemple les protéines de lait comme le lactalbumine, les protéines végétales telles que la protéine de soja vendue sous le nom d'Eleseryl par la société LSN ou le dérivé d'avoine vendu sous la dénomination « Reductine » par la société Silab, les acides nucléiques comme l'ADN.

Les compositions selon l'invention trouvent une application notamment comme compositions cosmétiques ou pharmaceutiques pour la peau, les muqueuses et/ou les semi-muqueuses.
Elles trouvent une application toute particulière en tant que produit de protection ou de soin de la peau du visage, du cou, des mains ou du corps, notamment en tant que composition anti-rides, anti-fatigue permettant de donner un coup d'éclat à la peau. On peut également envisager une application dans le domaine des compositions de maquillage de la peau du visage ou du corps, telles que les rouges à lèvres, les fonds de teint, les crèmes teintées, les sticks anti-cernes; ou les compositions anti-solaires ou de bronzage artificiel.
La composition de l'invention constitue plus particulièrement une composition antirides pour la peau du visage et/ou du cou.

L'invention est illustrée plus en détail dans les exemples suivants.

### Exemple 1

Préparation du composé de formule (I) dans laquelle :
R₁ = CH₃, X = O, R₂ = -CH₂-C₆H₄-pOH, R₃ = H et n =100 (indice théorique)

Dans un ballon de 500 ml, sous agitation, on introduit 20 g (0,096 mole) de N-carboxyanhydride tyrosine, 0,51 g (0,003 mole) de méthylate de sodium dans le méthanol, et 200 ml de tétrahydrofuranne anhydre.
On mélange vivement pendant 6 heures à 60°C. Un dégagement important de CO₂ se produit. A la fin de la réaction, on ajoute 10 ml d'eau. On évapore les solvants sous pression réduite.
On obtient 15 g de poudre de couleur jaune, soit un rendement de 96%.

### Exemple 2

Préparation du composé formule (I) dans laquelle :
R₁ = H, X = O, R₂ = H, R₃ = CH₃, R'₂ = -CH₂-C₆H₄-pOH et R'₃ = H.

Dans un ballon de 25 ml, on introduit 1,34 g (6,5 mmole) de N-carboxyanhydride tyrosine, 0,083 g (0,72 mmole) de N-carboxyanhydride sarcosine, 10 ml de dioxane anhydre et 0,043 ml d'une solution de triéthanolamine à 0,03% dans le dioxane, sous agitation. On chauffe à 37°C pendant 24 heures. Un dégagement important de CO₂ se produit. On évapore le solvant sous pression réduite.
On obtient 1,3 g de poudre de couleur jaune.
Le rapport théorique de motifs sarcosine par rapport aux motifs tyrosine est de 9.

### Exemple 3

Préparation du composé formule (I) dans laquelle :
R₁ = -(CH₂)₄-CH(NH₂)-COOH, X = NH, R₂ = H, R₃ = CH₃, R'₂ = -CH₂-C₆H₄-pOH et R'₃ = H.

Dans un ballon de 100 ml muni d'un réfrigérant et d'un bulleur, on introduit 4,5 g (22,7 mmole) de N-carboxyanhydride tyrosine, 0,5 g (4,5 mmole) de N-carboxyanhydride sarcosine, 50 ml de tétrahydrofuranne anhydre et 1,5 ml (0,21 mmol) d'une solution de L-lysine à 1% dans le dioxane, sous vive agitation.
On chauffe au reflux du THF pendant 6 heures. Un dégagement important de CO₂ se produit. Après retour à 20°C, on précipite le tout avec 50 ml d'acétate d'éthyle sous agitation, puis on filtre sur fritté n°3 et on sèche sous vide à 40°C.
On obtient 3,7 g de poudre de couleur blanche.
Le rapport théorique de motifs tyrosine par rapport aux motifs sarcosine est de 5.

### Exemple 4 : Crème antirides

On prépare une émulsion eau-dans-huile comprenant (% en poids):
*Phase A*
   - Polyisobutène hydrogéné 5,5%
   - Neopentanoate d'isostéaryle 3,5%
   - Stéarate de PEG-20 1%
   - Stéarate de glycérol + stéarate de PEG-100 2%
   - Alcool cétylique 0,5%
   - Alcool stéarylique 0,5%
   - Acide stéarique 1%
*Phase B*
   - Cyclométhicone 11%
*Phase C*
   - polyacrylamide + isoparaffine C13-14 + laureth-7 1%
*Phase D*
   - composé de l'exemple 1 7%
   - eau 25%
*Phase E*
   - conservateurs qs
   - triéthanolamine 0,03%
   - Eau déminéralisée qsp 100%

On chauffe la phase A sous agitation jusqu'à homogénéité. Après refroidissement, on ajoute la phase B. On chauffe la phase E sous agitation, puis on verse E dans A toujours sous agitation. Après refroidissement à 50°C, on incorpore la phase C à l'émulsion, puis la phase D.
On obtient une émulsion eau-dans-huile susceptible d'être employée comme crème anti-rides

### Exemple 5 : Sérum anti-vieillissement

On prépare un sérum en mélangeant (% en poids) :
- polyacrylamide + isoparaffine C13-14 + laureth-7 1%
- gomme xanthane 0,2%
- PVM/MA décadiène crosspolymer 0,2%
- triéthanolamine 0,2%
- composé de l'exemple 1 3,5%
- conservateurs qs
- eau qsp 100%

### Exemple 6 : Evaluation de l'effet tenseur par mesure au Dermomètre

On évalue l'effet 'tenseur' des composés selon l'invention par mesure au dermomètre. Cet appareil a été décrit par L. Rasseneur et al. dans Influence des Différents Constituants de la Couche Cornée sur la Mesure de son Elasticité, International Journal of Cosmetic Science, 4, 247-260 (1982). Le principe consiste à mesurer avant traitement et après traitement la longueur d'une éprouvette de stratum cornéum isolé et de déterminer le pourcentage de rétraction de l'éprouvette.

On utilise des éprouvettes de 0,6 cm x 0,4 cm de stratum cornéum d'épaisseur allant de 10 à 20 µm disposées sur l'extensiomètre MTT 610 commercialisé par la société DIASTRON.
L'éprouvette est placée entre 2 mâchoires puis laissée pendant 12 heures dans une atmosphère à 30°C et 40% d'humidité relative. Ces mâchoires sont alors fixées sur le Dermomètre.
On tracte à la vitesse de 1 mm/minute l'éprouvette d'une longueur comprise entre 5 et 10 % de la longueur initiale pour déterminer la longueur L₀ à partir de laquelle l'éprouvette commence à exercer une force sur les mâchoires et détectée par l'appareil.
On détend ensuite l'éprouvette puis on applique sur le stratum cornéum 2 mg de la composition à tester.
Pour préparer ces compositions, les composés sont solubilisés à chaud (60°C) dans le DMF, à une concentration de 7% en poids; la solution est maintenue à 60°C jusqu'à solubilisation complète. Elle est refroidie à 30°C avant utilisation.

On applique donc sur l'éprouvette la composition. Après évaporation totale de la composition (30 minutes de séchage), on tracte l'éprouvette dans les mêmes conditions que celles décrites précédemment pour déterminer également la longueur L₁ pour l'éprouvette traitée.

Le pourcentage de rétraction est déterminé par le rapport : 100 x (L₁-Lₒ)/Lₒ
Pour caractériser un effet tenseur, ce pourcentage doit être négatif et l'effet tenseur est d'autant plus important que la valeur absolue du pourcentage de rétraction est élevée.

On obtient les résultats suivants (moyenne et écart-type sur 7 échantillons) :

| Variation de la longueur de l'échantillon de stratum corneum en % et cinétique de l'effet : | | | | | | |
|---|---|---|---|---|---|---|
| Composé | 1H | 2H | 3H | Std-1 H | Std-2H | Std-3H |
| Exemple 1 | -0,9 | -1,6 | -1,5 | 1,4 | 1,1 | 1,1 |
| Exemple 2 | -1,3 | -1,1 | -0,9 | 0,7 | 0,7 | 0,5 |
| Exemple 3 | -1,5 | -1,1 | -0,5 | 1,4 | 1,0 | 1,1 |

## Revendications

1. Composition antirides comprenant, dans un milieu physiologiquement acceptable, au moins un composé de formule (I) : 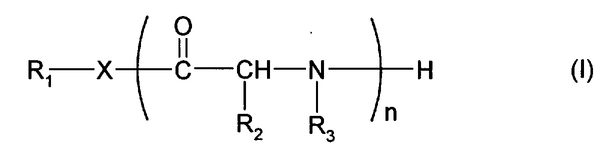 dans laquelle :
- X est choisi parmi -O-, -S- ou -NR, avec R représentant un atome d'hydrogène ou un radical hydrocarboné, linéaire ou ramifié, saturé ou insaturé en C₁-C₆,
- R₁ est choisi parmi :
(i) un atome d'hydrogène,
(ii) un radical hydrocarboné, linéaire ou ramifié, saturé ou insaturé, en C₁-C₄₀, éventuellement substitué par au moins un hydroxy ou un radical -NR'R", dans lequel R' et R" représentent, indépendamment l'un de l'autre, un atome d'hydrogène ou un radical hydrocarboné, linéaire ou ramifié, saturé ou insaturé en C₁-C₆; et/ou éventuellement interrompu par au moins un hétéroatome choisi parmi N, O ou Si,
(iii) un radical choisi parmi 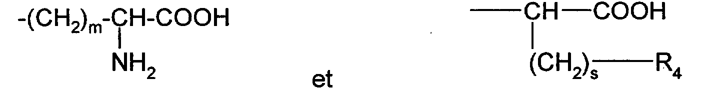 dans lesquels :
- m est 1, 2, 3, 4 ou 5;
- s est un entier compris entre 0 et 4 inclus;
- R₄ représente un atome d'hydrogène, -NH₂, -OH, -SH, -CHOHCH₃, -CONH₂, 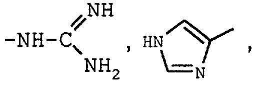 -C₆H₅ ou -C₆H₅pOH,
- R₂ représente un atome d'hydrogène, un radical hydrocarboné, linéaire ou ramifié, saturé ou insaturé, en C₁-C₈, ou un radical choisi parmi -CH₂OH, -CHOH-CH₃ , -CH₂C₆H₅, -CH₂C₆H₄p-OH et -(CH₂)ₜ-NH₂, avec t étant 1, 2, 3, 4 ou 5;
- R₃ représente un atome d'hydrogène ou un radical hydrocarboné, linéaire ou ramifié, saturé ou insaturé, en C₁-C₆,
- n est un nombre moyen d'unités répétitives supérieur à 1, et tel que le poids moléculaire du dérivé de polyaminoacide est compris entre 200 et 200.000, l'unité répétitive étant soit identique pour un même composé, soit différente, R₂ et/ou R₃ prenant alors au moins l'une des autres significations données pour ces radicaux, ou un de ses sels minéraux ou organiques.

2. Composition selon la revendication 1, dans laquelle X représente O, S ou N-CH₃.

3. Composition selon l'une des revendications précédentes, dans laquelle R₁ représente un atome d'hydrogène; un radical hydrocarboné, linéaire ou ramifié, saturé ou insaturé, en C₁-C₂₂, notamment en C4-20, ou un radical choisi parmi : 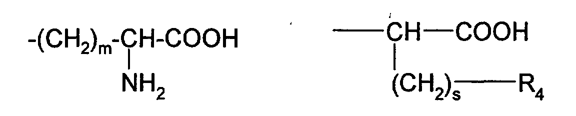 dans lesquels m, s et R4 ont les significations données ci-dessus.

4. Composition selon l'une des revendications précédentes, dans laquelle R₂ représente un atome d'hydrogène, un radical hydrocarboné, linéaire ou ramifié, saturé ou insaturé, en C₁-C₆, ou un radical -CH₂C₆H₄p-OH.

5. Composition selon l'une des revendications précédentes, dans laquelle R₃ représente un atome d'hydrogène ou un radical hydrocarboné, linéaire ou ramifié, saturé ou insaturé, en C₁-C₄, notamment méthyle bu éthyle.

6. Composition selon l'une des revendications précédentes, dans laquelle R₁ représente l'une des formules suivantes :
C₁₅H₃₁-CH(OH)-CH(CH₂OH)-
C₁₀H₂₁-CH(C₈H₁₇)-CH₂-
C₁₆H₃₃-
C₈H₁₇-CH=CH-C₈H₁₆- 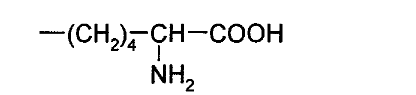 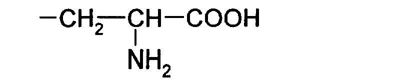 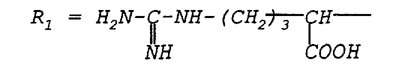 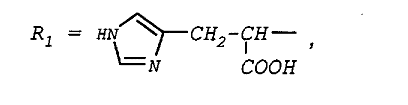 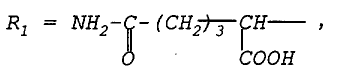
CH₂(OH)-CH(OH)-CH(OH)-CH(OH)-CH(OH)-CH₂-

7. Composition selon l'une des revendications précédentes, dans laquelle n est compris entre 3 et 500 et/ou est tel que le poids moléculaire du dérivé de polyaminoacide est compris entre 300 et 50 000.

8. Composition selon l'une des revendications précédentes, dans laquelle les composés de formule (I) sont présents, seuls ou en mélange, en une quantité de 0,001 à 30% en poids par rapport au poids total de la composition, de préférence en une quantité de 0,01 à 15% en poids par rapport au poids total de la composition.

9. Composition selon l'une des revendications précédentes, se présentant sous la forme d'une composition cosmétique ou pharmaceutique comprenant un milieu cosmétiquement ou pharmaceutiquement acceptable.

10. Composition selon l'une des revendications précédentes, comprenant en outre des agents tenseurs tels qu'une protéine ou hydrolysat de protéine, et notamment les protéines de lait comme le lactalbumine, les protéines végétales telles que la protéine de soja et le dérivé d'avoine ; les acides nucléiques comme l'ADN.

11. Composition selon l'une des revendications précédentes, se présentant sous forme d'une solution aqueuse, hydroalcoolique ou huileuse; d'une émulsion huile-dans-eau, eau-dans-huile ou multiple, de consistance liquide ou semi-liquide du type lait, ou de consistance molle, semi-solide ou solide du type crème; d'un gel aqueux ou huileux; d'un produit anhydre liquide, pâteux ou solide; de dispersions aqueuses, huileuses ou en milieu solvant de type lotion ou sérum; de microémulsions; de microcapsules; de microparticules ou de dispersions vésiculaires de type ionique ou non ionique; sous forme fluide, épaissie ou gélifiée, semi-solide, pâte souple; sous forme solide telle que de stick ou bâton ; d'une crème blanche ou colorée, d'une pommade, d'un lait, d'une lotion, d'un sérum, d'une pâte, d'une mousse ; d'aérosol ; sous forme solide, et par exemple sous forme de stick.

12. Composition selon l'une des revendications précédentes, se présentant sous la forme d'un produit de protection ou de soin de la peau du visage, du cou, des mains ou du corps, notamment en tant que composition anti-rides, anti-fatigue permettant de donner un coup d'éclat à la peau ; ou sous la forme d'une composition de maquillage de la peau du visage ou du corps, telle qu'un rouge à lèvres, un fond de teint, une crèmes teintée, un stick anti-cernes; une composition anti-solaires ou de bronzage artificiel.

13. Composition selon l'une des revendications précédentes, se présentant sous la forme d'une composition antirides pour la peau du visage et/ou du cou.

14. Utilisation d'au moins un composé de formule (I) tel que défini dans l'une des revendications 1 à 7, ou d'une composition cosmétique le comprenant, pour diminuer, effacer et/ou lisser les rides et/ou les ridules de la peau.

15. Utilisation d'au moins un composé de formule (I) tel que défini dans l'une des revendications 1 à 7, pour la fabrication d'une composition pharmaceutique destinée à diminuer, effacer et/ou lisser les rides et/ou les ridules de la peau.

16. Utilisation d'au moins un composé de formule (I) tel que défini dans l'une des revendications 1 à 7, ou d'une composition le comprenant, comme agent tenseur pour diminuer, lisser et/ou effacer les rides et/ou les ridules de la peau.

17. Procédé de traitement cosmétique d'une peau ridée consistant à appliquer sur la ride et/ou la ridule, une composition comprenant au moins un composé de formule (I) tel que défini dans l'une des revendications 1 à 7.
